(19) Europäisches Patentamt
European Patent Office
Office européen des brevets

(11) Publication number: 0 415 304 A2

(12) **EUROPEAN PATENT APPLICATION**

(21) Application number: 90116338.6

(22) Date of filing: 27.08.90

(51) Int. Cl.5: C07D 498/14, C07D 513/14, A61K 31/55, //(C07D498/14, 267:00,221:00,221:00), (C07D513/14,281:00,221:00, 221:00)

(30) Priority: 29.08.89 US 399802
18.04.90 US 510642

(43) Date of publication of application:
06.03.91 Bulletin 91/10

(84) Designated Contracting States:
AT BE CH DE DK ES FR GB GR IT LI LU NL SE

(71) Applicant: Boehringer Ingelheim Pharmaceuticals Inc.
90 East Ridge P.O. Box 368
Ridgefield Connecticut 06877(US)

(72) Inventor: Hargrave, Karl D. Dr.
4, Edna Drive Brookfield
Connecticut 06805(US)

(74) Representative: Laudien, Dieter, Dr. et al
Boehringer Ingelheim GmbH, Abteilung Patente
W-6507 Ingelheim am Rhein(DE)

(54) **Dipyrido[3,2-b:2',3'-e][1,4]oxazepin (and thiazepin)-6(5H)-ones and -thiones and their use in the prevention or treatment of aids.**

(57) Disclosed are dipyrido[3,2-b:2′,3′-e][1,4]oxazepin (and thiazepin)-6(5H)-ones and -thiones. These are useful in the prevention or treatment of AIDS.

EP 0 415 304 A2

# DIPYRIDO[3,2-B:2′,3′-E][1,4]OXAZEPIN (AND THIAZEPIN)-6(5H)-ONES AND -THIONES AND THEIR USE IN THE PREVENTION OR TREATMENT OF AIDS

## Related Applications

This is a continuation-in-part of application serial number 399,802, filed August 29, 1989.

## Field of the Invention

The invention relates to novel dipyrido[3,2-b:2′,3′-e][1,4]oxazepin (and thiazepin)-6(5H)-ones and -thiones and pharmaceutically acceptable acid addition salts thereof, methods for preparing these compounds, the use of these compounds in the prevention or treatment of AIDS, and to pharmaceutical compositions containing these compounds.

## Background of the Invention

The human disease, Acquired Immune Deficiency Syndrome (AIDS), is caused by the Human Immunodeficiency Virus (HIV), particularly the strain known as HIV-1.

Like other viruses, HIV-1 cannot replicate without commandeering the biosynthetic apparatus or the host cell it infects. It causes this apparatus to produce the structural proteins which make up the viral progeny. These proteins are coded for by the genetic material contained within the infecting virus particle, or virion. Being a retrovirus, however, the genetic material of HIV is RNA, not DNA as in the host cell's genome. Accordingly, the viral RNA must first be converted into DNA, and then integrated into the host cell's genome, in order for the host cell to produce the required viral proteins.

The conversion of the RNA to DNA is accomplished through the use of the enzyme reverse transcriptase (RT), which is included within the infecting virion along with the RNA. Reverse transcriptase has three enzymatic functions; it acts as an RNA-dependent DNA polymerase, as a ribonuclease, and as a DNA-dependent DNA polymerase. Acting first as an RNA-dependent DNA polymerase, RT makes a single-stranded DNA copy of the viral RNA. Next, acting as a ribonuclease, RT frees the DNA just produced from the original viral RNA and then destroys the original RNA. Finally, acting as a DNA-dependent DNA polymerase, RT makes a second, complementary DNA strand, using the first DNA strand as a template. The two strands form double-stranded DNA, which is integrated into the host cell's genome by another enzyme, called an integrase.

Compounds which inhibit the enzymatic functions of HIV-1 reverse transcriptase will inhibit replication of HIV-1 in infected cells. Such compounds are useful in the prevention or treatment of HIV-1 infection in human subjects.

## Description of the Invention

In its broadest composition of matter aspect, the invention comprises dipyrido[3,2-b:2′,3′-e][1,4]-oxazepin (and thiazepin)-6(5H)-ones and -thiones of the formula I

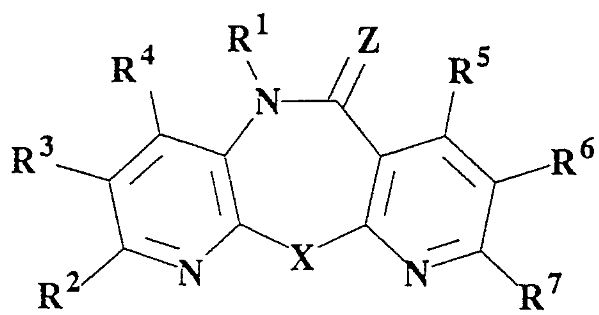

(I)

wherein,
X is oxygen or sulfur;
Z is oxygen or sulfur;
$R^1$ is alkyl of 1 to 5 carbon atoms, cycloalkyl of 3 to 5 carbon atoms, fluoroalkylmethyl of 1 to 3 fluorine atoms and 2 to 4 carbon atoms, mono- or dihaloalkenyl of 2 to 4 carbon atoms wherein the halogen atoms are attached to the vinylic carbon atoms, alkenyl or alkynyl of 2 to 4 carbon atoms, alkyloxyalkyl or alkylthioalkyl of 2 to 4 carbon atoms, aminocarbonylmethyl, acetyl, cyanoalkyl wherein the alkyl moiety contains 1 to 3 carbon atoms, or hydroxyalkylmethyl of 2 to 4 carbon atoms;
$R^2$ is hydrogen, methyl, ethyl, halogen, nitro or amino;
$R^3$ is hydrogen, methyl or halogen;
$R^4$ is hydrogen, alkyl of 1 to 4 carbon atoms, alkenyl or alkynyl of 2 to 3 carbon atoms, trihalomethyl, alkanoyl of 2 to 3 carbon atoms, cyano, azido, amino, nitro, halogen, hydroxyl, alkyloxy or alkylthio of 1 to 2 carbon atoms, monk or di-alkylamino wherein each alkyl group contains 1 to 2 carbon atoms, aminoalkyl or mono- or di-alkylaminoalkyl wherein each alkyl group contains 1 to 2 carbon atoms, hydroxyalkyl of 1 to 3 carbon atoms or alkyloxycarbonyl of 2 to 3 carbon atoms;
with the proviso that when $R^4$ is other than hydrogen, $R^2$ is hydrogen, methyl or chloro and $R^3$ is hydrogen;
$R^5$ is hydrogen, methyl or halogen;
$R^6$ is hydrogen, methyl, halogen or amino; and,
$R^7$ is hydrogen, methyl or halogen;
with the proviso that at least two of $R^5$, $R^6$ and $R^7$ is hydrogen.

A subgeneric aspect of the invention comprises compounds of formula I, wherein
X is oxygen or sulfur;
Z is oxygen;
$R^1$ is alkyl of 1 to 4 carbon atoms, fluoroalkylmethyl of 1 to 3 fluorine atoms and 2 to 4 carbon atoms, mono- or dihalovinyl, 2-chloro-2-propen-1-yl, alkenylmethyl or alkynylmethyl of 3 to 4 carbon atoms, or alkyloxyalkyl or alkylthioalkyl of 2 to 4 carbon atoms;
$R^2$ is hydrogen, methyl, ethyl or chloro;
$R^3$ is hydrogen;
$R^4$ is hydrogen, methyl, ethyl, chloro, fluoro, vinyl, trifluoromethyl, acetyl or cyano; and,
$R^5$, $R^6$ and $R^7$ are each hydrogen or methyl, with the proviso that at least two of them are hydrogen.

A particular subgeneric aspect of the invention comprises compounds of formula I, wherein
X is oxygen or sulfur;
Z is oxygen;
$R^1$ is alkyl of 1 to 3 carbon atoms, allyl, or alkyloxyalkyl or alkylthioalkyl of 2 to 3 carbon atoms;
$R^2$ is hydrogen, methyl or chloro;
$R^3$ is hydrogen;
$R^4$ is hydrogen, methyl, ethyl, chloro or trifluoromethyl; and,
$R^5$, $R^6$ and $R^7$ are hydrogen.

The compounds of formula I can be prepared by known methods or obvious modifications thereof. Methods A,B,C and D, described below, are illustrative of methods for preparing compounds of formula I.

Method A

Compounds of formula I, wherein Z is oxygen and $R^1$ through $R^7$ have the meanings given above, may be obtained, for example, by converting a compound of the formula II

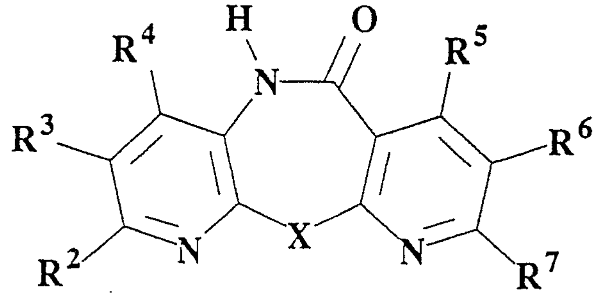

(II)

wherein $R^2$ through $R^7$ are as defined above, into the corresponding alkali or alkaline earth metal compounds of the formula III

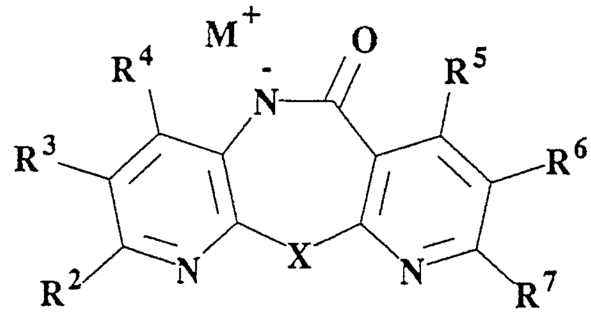

(III)

wherein $R^2$ through $R^7$ are as defined above, and subsequently reacting, without isolation, this alkali metal compound with a reactive alkylating or acylating reagent of the formula IV

$R^1Y$ (IV)

wherein $R^1$ has the meanings defined above, and Y is a suitable leaving group such as chloride, bromide, iodide, an alkyl- or arylsulfonate, or an alkyl- or arylcarbonyloxy group, under well known alkylating or acylating conditions.

It will be obvious to those skilled in the art that the presence of nucleophilic substituent in the compound of formula II will require the use of an intermediate having a substituent which is, other than the 5-position nitrogen, not nucleophilic but which can be derivatized to yield the required group. For example, amino or monoalkylamino substituents are preferably obtained by alkylating or acylating an intermediate of formula II having nitro group(s) at the desired positions, and subsequently reducing the nitro group(s), and alkylating, if appropriate, to yield the final product.

Method B

Compounds of formula II wherein Z is oxygen and X and $R^2$ through $R^7$ are as defined above may be obtained by cyclization of compounds of the formula V

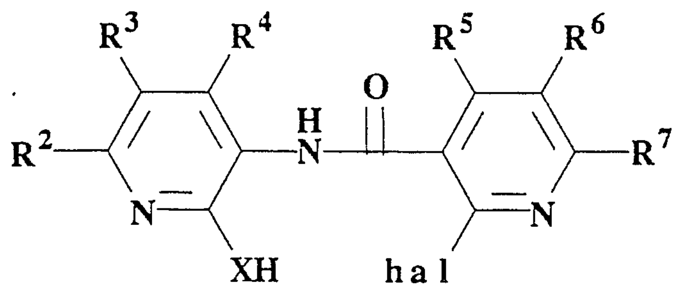

(V)

wherein X and $R^2$ through $R^7$ are as defined above and hal is fluorine, chlorine, bromine or iodine, preferably in the presence of an inorganic base, such as sodium or potassium hydride, lithium alkyls such as n-butyl lithium sodium or potassium hydroxide, or in the presence of an organic base, such as quinoline or 4-(N,N-dimethylamino)pyridine, at ambient or elevated temperatures, preferably 80-175 °C, up to the boiling point of the reaction mixture. Suitable solvents include inert aprotic solvents such as toluene, sulfolane or dimethylformamide.

The dipyridinylamides of formula V may be obtained, for example, by condensing suitably substituted 2-halonicotinic acid chlorides of the formula VI

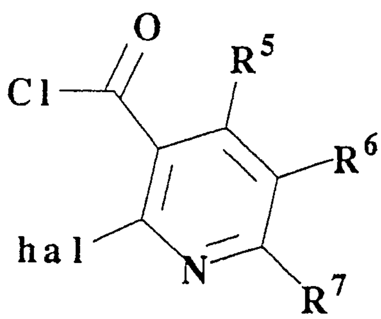

(VI)

wherein hal may be fluorine, chlorine, bromine or iodine and $R^5$ through $R^7$ are as defined above, with 2-hydroxy (or mercapto)-3-aminopyridines the formula VII

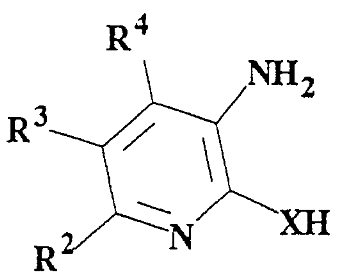

(VII)

wherein X and $R^2$ through $R^4$ are as defined above, under well-known reaction conditions. Depending upon the reaction conditions and the nature of X and $R^2$ through $R^7$, tricyclic compounds of the formula II, wherein X and $R^2$ through $R^7$ are as defined above, may be formed in one step, without the isolation of the amide of formula V, by the condensation of compounds of the formulas VI and VII. This single-step formation of the tricyclic compound is most readily effected when X is sulfur and at elevated temperatures, especially in the range of 125-200 °C.

Method C

An alternative procedure to obtain compounds of the formula II, wherein Z is oxygen and X and $R^2$ through $R^7$ are as defined above, is to condense, suitably substituted 2-hydroxy (or mercapto) nicotinic acids of the formula VIII

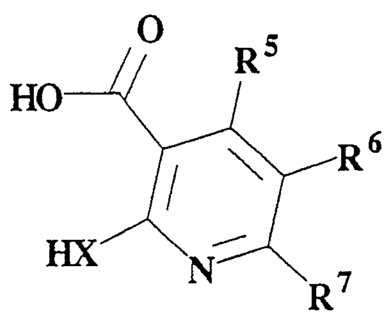

(VIII)

wherein X and $R^5$ through $R^7$ are as defined above, with suitably substituted 2-halo-3-aminopyridines of the formula IX

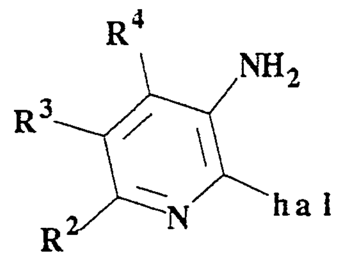

(IX)

wherein hal is fluorine, chlorine, bromine or iodine and $R^2$ through $R^4$ are as defined above. The reaction is performed under an inert atmosphere, such as nitrogen, at an elevated temperature, preferably at 150-225 °C, in an inert solvent such as trichlorobenzene.

Method D

Thiolactams of formula I, wherein Z is sulfur and X and $R^1$ through $R^7$ are as defined above, can be obtained by treatment of lactams of the formula I with sulfurating reagents such as 2,4-bis(4-methoxyphenyl)-1,3-dithia-2,4-diphosphetane-2,4-disulfide, bis(tricyclohexyltin)sulfide, bis(tri-n-butyltin) sulfide, bis-(triphenyltin)sulfide, bis(trimethylsilyl)sulfide and phosphorous pentasulfide. The reaction is generally carried out preferably under anhydrous conditions in inert organic solvents such as carbon disulfide, benzene, or toluene, for example at room temperature or, preferably, higher temperatures up to the boiling point of the reaction mixture. When using the above mentioned tin or silyl sulfides it is preferable to carry out the sulfurization reaction in the presence of a Lewis acid such as boron trichloride.

Compounds of formula I may, if desired, be converted into their non-toxic, pharmaceutically acceptable acid addition salts by conventional methods; for example, by dissolving a compound of formula I in a suitable solvent and acidifying the solution with one or more molar equivalents of the desired acid. The invention comprises such salts.

Examples of inorganic and organic acids which may form nontoxic, pharmaceutically acceptable acid addition salts with a compound of the formula I are the following: hydrochloric acid, hydrobromic acid, sulfuric acid, phosphoric acid, nitric acid, tartaric acid, citric acid, methanesulfonic acid, and the like.

The above described compounds of Formula I possess inhibitory activity against HIV-1 reverse transcriptase. When administered in suitable dosage forms, they are useful in the prevention or treatment of AIDS, ARC and related disorders associated with HIV-1 infection. Another aspect of the invention, therefore, is a method for preventing or treating HIV-1 infection which comprises administering, to a human being exposed to or infected by HIV-1, a prophylactically or therapeutically effective amount of a novel compound of Formula I, as described above.

The compounds of formula I may be administered in single or divided doses by the oral, parenteral or topical routes. A suitable oral dosage for a compound of formula I would be in the range of about 10 to 500 mg per day. In parenteral formulations, a suitable dosage unit may contain from 1 to 50 mg of said compounds, whereas for topical administration, formulations containing 0.01 to 1% active ingredient are preferred. It should be understood, however, that the dosage administration from patient to patient will vary and the dosage for any particular patient will depend upon the clinician's judgement, who will use as criteria for fixing a proper dosage the size and condition of the patient as well as the patient's response to the drug.

When the compounds of the present invention are to be administered by the oral route, they may be administered as medicaments in the form of pharmaceutical preparations which contain them in association with a compatible pharmaceutical carrier material. Such carrier material can be an inert organic or inorganic carrier material suitable for oral administration. Examples of such carrier materials are water, gelatin, talc, starch, magnesium stearate, gum arabic, vegetable oils, polyalkylene-glycols, petroleum jelly and the like.

The pharmaceutical preparations can be prepared in a conventional manner and finished dosage forms can be solid dosage forms, for example, tablets, dragees, capsules, and the like, or liquid dosage forms, for example solutions, suspensions, emulsions and the like. The pharmaceutical preparations may be subjected to conventional pharmaceutical operations such as sterilization. Further, the pharmaceutical preparations may contain conventional adjuvants such as preservatives, stabilizers, emulsifiers, flavor-improvers, wetting agents, buffers, salts for varying the osmotic pressure and the like. Solid carrier material which can be used include, for example, starch, lactose, mannitol, methyl cellulose, microcrystalline cellulose, talc, silica, dibasic calcium phosphate, and high molecular weight polymers (such as polyethylene glycol).

For parenteral use, a compound of formula I can be administered in an aqueous or non-aqueous

solution, suspension or emulsion in a pharmaceutically acceptable oil or a mixture of liquids, which may contain bacteriostatic agents, antioxidants, preservatives, buffers or other solutes to render the solution isotonic with the blood, thickening agents, suspending agents or other pharmaceutically acceptable additives. Additives of this type include, for example, tartrate, citrate and acetate buffers, ethanol, propylene glycol, polyethylene glycol, complex formers (such as EDTA), antioxidants (such as sodium bisulfite, sodium metabisulfite, and ascorbic acid), high molecular weight polymers (such as liquid polyethylene oxides) for viscosity regulation and polyethylene derivatives of sorbitol anhydrides. Preservatives may also be added if necessary, such as benzoic acid, methyl or propyl paraben, benzalkonium chloride and other quaternary ammonium compounds.

The compounds of the invention may also be administered as solutions for nasal application and may contain in addition to the compounds of this invention suitable buffers, tonicity adjusters, microbial preservatives, antioxidants and viscosity-increasing agents in an aqueous vehicle. Examples of agents used to increase viscosity are polyvinyl alcohol, cellulose derivatives, polyvinylpyrrolidone, polysorbates or glycerin. Microbial preservatives added may include benzalkonium chloride, thimerosal, chlorobutanol or phenylethyl alcohol.

Additionally, the compounds provided by the invention can be administered by suppository.

As stated before, the described compounds provided by the invention inhibit the enzymatic activity of HIV-1 RT. Based upon testing of these compounds, as described below, it is known that they inhibit the RNA-dependent DNA polymerase activity of HIV-1 RT. It is believed that they also inhibit the DNA-dependent DNA polymerase activity of HIV-1 RT.

Utilizing the Reverse Transcriptase (RT) Assay described below, compounds can be tested for their ability to inhibit the RNA-dependent DNA polymerase activity of HIV-1 RT. The compound described in Example 1, which appears below, was so tested. This compound was found to inhibit (by 30%) the enzyme when tested at a concentration of 10μg/ml.

## REVERSE TRANSCRIPTASE (RT) ASSAY

Assay Theory:

Among the enzymes for which Human Immunodeficiency Virus (HIV-1) encodes is a reverse transcriptase (1), so-named because it transcribes a DNA copy from an RNA template. This activity can be quantitatively measured in a cell-free enzyme assay which has been previously described (2), and is based upon the observation that reverse transcriptase is able to use a synthetic template [poly r(C) primed with oligo d(G)] to transcribe a radio-labelled, acid-precipitable DNA strand utilizing $^{3}$H-dGTP as a substrate.

Materials:

a) Preparation of the enzyme

Reverse transcriptase enzyme from the LAV strain of Human Immunodeficiency Virus (HIV-1) (1) was isolated from the bacterial strain JM109 (3) expressing the DNA clone pBRTprtl+ (2) which is under the control of the lac promotor in the expression vector pIB121 (4). An overnight culture grown in 2XYT medium (37° C, 225 rpm) (5) supplemented with 100 μg/ml ampicillin for positive selection is inoculated at a 1:40 dilution into M9 medium supplemented with 10μg/ml thiamine, 0.5% casamino acids, and 50 μg/ml ampicillin (5). The culture is incubated (37° C, 225 rpm) until it reaches an OD540 of 0.3-0.4. At that time the repressor inhibitor IPTG (isopropyl β-D-thiogalactopyranoside) is added to 0.5 mM, and the mixture incubated for 2 additional hours. Bacteria are pelleted, resuspended in a 50 mM Tris, 0.6mM EDTA, 0.375 M NaCl buffer and digested by the addition of lysozyme (1 mg/ml) for 30 minutes on ice. The cells are lysed by the addition to 0.2% NP-40 and brought to 1 M NaCl.

After removal of the insoluble debris by centrifugation, the protein is precipitated by the addition of 3 volumes of saturated aqueous ammonium sulfate. The enzyme is pelleted, resuspended in RT buffer (50 mM Tris pH 7.5,1 mM EDTA, 5 mM DTT, 0.1% NP-40, 0.1 M NaCl, and 50% glycerol), and stored at -70° C for further use.

| b) Composition of 2X concentrated stock reaction mixture | |
|---|---|
| Stock Reagent | 2X Mix Concentration |
| 1M Tris pH 7.4 | 100 mM |
| 1M Dithiothrietol | 40 mM |
| 1M NaCl | 120 mM |
| 1% Nonidet P-40 | 0.1% |
| 1M $MgCl_2$ | 4 mM |
| [poly r(C) /oligo d(G)](5:1) | 2 µg/ml |
| $^3$H-dGTP (81 µM) | 0.6 µM |

Assay Procedure:

The 2X concentrated stock reaction mixture is aliquoted and stored at -20° C. The mixture is stable and thawed for use in each assay. This enzyme assay has been adapted to a 96 well microtiter plate system, and has been previously described (6). Tris buffer (50 mM, pH 7.4), vehicle (solvent diluted to match the compound dilution), or compounds in vehicle are dispensed into 96-well microtiter plates (10µl/well; 3 wells/compound). The HIV-1 RT enzyme is thawed, diluted in 50 mM Tris pH 7.4 so that fifteen µl of diluted enzyme contain 0.001 Unit (one unit is that amount of enzyme to transform 1 micromole of substrate per minute at 25° C), and fifteen µl are dispensed per well. Twenty µl of 0.12-0.5 M EDTA are added to the first three wells of the microtiter plate. EDTA chelates the $Mg^{++}$ present and prevents reverse transcription. This group serves as background polymerization which is subtracted from all other groups. Twenty-five µl of the 2X reaction mixture are added to all wells and the assay is allowed to incubate at room temperature for 60 minutes. The assay is terminated by precipitating the DNA in each well with 50 µ l of trichloracetic acid (TCA) (10% w/v) in sodium pyrophosphate (1% w/v). The microtiter plate is incubated for 15 minutes at 4° C and the precipitate is fixed onto #30 glass fiber paper (Schleicher & Schuell) using a Skatron semi-automatic harvester. The filters are then washed with additional TCA (5%) containing sodium pyrophosphate (1%), rinsed with aqueous ethanol (70%), dried, and transferred to scintillation vials (6). Each vial receives 2 ml of scintillation cocktail and is counted in a Beckman beta counter.

Calculations for percent inhibition are as follows:

$$\%\text{inhibition} = \frac{\text{CPM Mean Test Value - CPM Mean Control Value X100}}{\text{CPM Mean Control Value}}$$

References:


1. Benn, S., et al., *Science* 230:949, 1985
2. Farmerie, W.G. et. al., *Science* 236:305, 1987
3. Yanisch-Perron, C., Viera, J., and Messing, J., *Gene* 33:103, 1985
4. International Biotechnologies, Inc., New Haven, CT 06535
5. Maniatis, T, Fritsch, E.F., and J. Sambrook, eds. *Molecular Cloning: A Laboratory Manual*, Cold Spring Harbor Laboratory, 1982
6. Spira, T., et. al., *J. Clinical Microbiology*, 25:97, 1987.


The following examples further illustrate the present invention and will enable others skilled in the art to understand it more completely. It should be understood, however, that the invention is not limited to the particular examples given below.

Example 1

5,6-Dihydro-5-propyl-6H-dipyrido[3,2-b:2′,3′-e][1,4]oxazepin-6-one

(a) 3-(2-Chloronicotinamido)-2-hydroxypyridine

A mixture of 2-chloronicotinic acid (3.9 g, 0.025 mol) and thionyl chloride (10mL, 0.14 mol) was heated at reflux for 1 hour to produce a clear yellow solution. Excess thionyl chloride was removed by rotary evaporation, and the residue was dissolved in 20 ml of tetrahydrofuran. The resulting solution was added slowly dropwise to a solution of 2-hydroxy-3-aminopyridine (2.75 g, 0.025 mol) and diisopropylethylamine (4.8 ml, 0.028 mol) in 100 ml of tetrahydrofuran at 0 °C under argon. After the addition was complete, the reaction mixture was allowed to warm to room temperature, and stirring was continued overnight.

The reaction mixture was diluted with a mixture of water and diethyl ether, producing emulsions and solid precipitation. Filtration provided a clean separation of layers and also afforded 3.4g (54%) of the desired amide as a tan powder, mp 214-215 °C. The organic extracts were concentrated, and the residue was stirred with hot ethyl acetate. Filtration afforded an additional 1.3 g (21%) of the product.

(b) 5,6-Dihydro-6H-dipyrido[3,2-b:2′,3′-e][1,4]oxazepin-6-one

Sodium hydride (50% dispersion in oil, 0.74 g, 0.015 mol) was added to 30 ml of absolute ethanol and to the resulting solution of sodium ethoxide in ethanol was added the amide prepared above (3.4 g, 0.015 mol). The mixture was stirred under argon at 50 °C for 45 min. Concentration by rotary evaporation then gave a brown oil. Dimethylformamide (30 ml) was added, and the reaction mixture was stirred overnight at 100 °C under argon. Stirring was then continued for an additional 4 hours at 140 °C.

After cooling to room temperature, the reaction mixture was poured into ice-water, diluted with additional water, and filtered to give a yellow-brown solid. After air-drying, the solid (2.4g) was stirred in 200 ml of hot ethyl acetate and filtered, collecting 0.89 g of a shiny brown powder, which was still impure by TLC. Stirring with 100 ml of hot ethanol and filtration afforded 0.39g (12%) of the pure oxazepinone product as a glistening off-white solid, mp>280 °C.

c) 5,6-Dihydro-5-propyl-6H-dipyrido[3,2-b:2′,3′-e][1,4]oxazepin-6-one

To a suspension of the oxazepinone (0.21 g, 1.0 mmol) in 7.5 ml of DMF was added sodium hydride (50% dispersion in oil, 0.07 g, 1.5 mmol), and the resulting mixture was stirred for 1 hour at 50 °C under argon. The nearly clear yellow solution was then cooled to room temperature, and 1-bromopropane (0.13 ml, 1.4 mmol) was added by syringe. Stirring was continued at room temperature overnight.

The reaction mixture was diluted with water and extracted with diethyl ether and ethyl acetate. The combined organic extracts were washed with brine, dried over magnesium sulfate, and concentrated to give a yellow liquid. Purification by short-path chromatography (elution with 20-50% ethyl acetate-hexane) afforded 0.13 g (51%) of the pure product as white crystals, mp 108-109.5 °C.

| EXAMPLE A | | | |
|---|---|---|---|
| Capsules or Tablets | | | |
| A-1 | A-2 | | |
| Ingredients | Quantity | Ingredients | Quantity |
| Compound of Example 1<br>Starch<br>Microcrys., Cellulose<br>Sodium Starch Glycolate<br>Magnesium Stearate<br>Fumed colloidal silica | 50 mg<br>160 mg<br>90 mg<br>10 mg<br>2 mg<br>1 mg | Example 1<br>Dicalcium Phosphate<br>Microcrys., Cellulose<br>Stearic acid<br>Sodium Starch Glycolate<br>Fumed colloidal silica | 50 mg<br>160 mg<br>90 mg<br>5 mg<br>10 mg<br>1 mg |

The compound of Example 1 is blended into a powder mixture with the premixed excipient materials as identified above with the exception of the lubricant. The lubricant is then blended in and the resulting blend compressed into tablets or filled into hard gelatin capsules.

| EXAMPLE B | |
|---|---|
| Parenteral Solution | |
| Ingredients | Quantity |
| Compound of Example 1<br>Tartaric acid<br>Benzyl Alcohol<br>Water for injection | 500 mg<br>1.5 g<br>0.1% by weight<br>q.s. to 100 ml |

The excipient materials are mixed with the water and thereafter the compound of Example 1 is added. Mixing is continued until the solution is clear. The pH of this solution is adjusted to 3.0 and is then filtered into the appropriate vials or ampoules and sterilized by autoclaving.

| EXAMPLE C | |
|---|---|
| Nasal Solution | |
| Ingredients | Quantity |
| Compound of Example 1<br>Citric acid<br>Benzalkonium chloride<br>EDTA<br>Polyvinylalcohol<br>Water | 100 mg<br>1.92 g<br>0.025% by weight<br>0.1% by weight<br>10% by weight<br>q.s. to 100 ml |

The excipient materials are mixed with the water and thereafter the compound of Example 1 is added and mixing is continued until the solution is clear. The pH of this solution is adjusted to 4.0 and is then filtered into the appropriate vials or ampoules.

## Claims

1. A compound of the formula I,

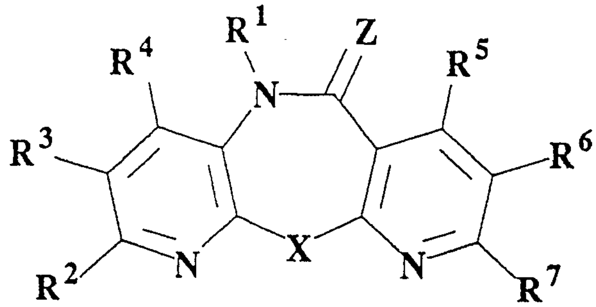

(I)

wherein,
X is oxygen or sulfur;
Z is oxygen or sulfur;
$R^1$ is alkyl of 1 to 5 carbon atoms, cycloalkyl of 3 to 5 carbon atoms, fluoroalkylmethyl of 1 to 3 fluorine atoms and 2 to 4 carbon atoms, mono- or dihaloalkenyl of 2 to 4 carbon atoms wherein the halogen atoms are attached to the vinylic carbon atoms, alkenyl or alkynyl of 2 to 4 carbon atoms, alkyloxyalkyl or alkylthioalkyl of 2 to 4 carbon atoms, aminocarbonylmethyl, acetyl, cyanoalkyl wherein the alkyl moiety contains 1 to 3 carbon atoms, or hydroxyalkylmethyl of 2 to 4 carbon atoms;
$R^2$ is hydrogen, methyl, ethyl, halogen, nitro or amino;
$R^3$ is hydrogen, methyl or halogen;
$R^4$ is hydrogen, alkyl of 1 to 4 carbon atoms, alkenyl or alkynyl of 2 to 3 carbon atoms, trihalomethyl, alkanoyl of 2 to 3 carbon atoms, cyano, azido, amino, nitro, halogen, hydroxyl, alkyloxy or alkylthio of 1 to 2 carbon atoms, mono or di-alkylamino wherein each alkyl group oontains 1 to 2 carbon atoms, aminoalkyl or mono- or di-alkylaminoalkyl wherein each alkyl group contains 1 to 2 carbon atoms, hydroxyalkyl of 1 to 3 carbon atoms or alkyloxycarbonyl of 2 to 3 carbon atoms;
with the proviso that when $R^4$ is other than hydrogen, $R^2$ is hydrogen, methyl or chloro and $R^3$ is hydrogen;
$R^5$ is hydrogen, methyl or halogen;
$R^6$ is hydrogen, methyl, halogen or amino; and,
$R^7$ is hydrogen, methyl or halogen;
with the proviso that at least two of $R^5$, $R^6$ and $R^7$ is hydrogen;
or a pharmaceutically acceptable salt thereof.

2. A compound of formula I, in accordance with claim 1, wherein,
X is oxygen or sulfur;
Z is oxygen;
$R^1$ is alkyl of 1 to 4 carbon atoms, fluoroalkylmethyl of 1 to 3 fluorine atoms and 2 to 4 carbon atoms, mono- or dihalovinyl, 2-chloro-2-propen-1-yl, alkenylmethyl or alkynylmethyl of 3 to 4 carbon atoms, or alkyloxyalkyl or alkylthioalkyl of 2 to 4 carbon atoms;
$R^2$ is hydrogen, methyl, ethyl or chloro;
$R^3$ is hydrogen;
$R^4$ is hydrogen, methyl, ethyl, chloro, fluoro, vinyl, trifluoromethyl, acetyl or cyano; and,
$R^5$, $R^6$ and $R^7$ are each hydrogen or methyl, with the proviso that at least two of them are hydrogen.

3. A compound of formula I, in accordance with claim 1, wherein,
X is oxygen or sulfur;
Z is oxygen;
$R^1$ is alkyl of 1 to 3 carbon atoms, alkyl, or alkyloxyalkyl or alkylthioalkyl of 2 to 3 carbon atoms;
$R^2$ is hydrogen, methyl or chloro;
$R^3$ is hydrogen;
$R^4$ is hydrogen, methyl, ethyl, chloro or trifluoromethyl; and,
$R^5$, $R^6$ and $R^7$ are hydrogen.

4. 5,6-Dihydro-5-propyl-6H-dipyrido[3,2-b:2′,3′-e][1,4]oxazepin-6-one, or a pharmaceutically acceptable salt thereof.

5. A method for preventing or treating HIV-1 infection which comprises administering, to a human who has been exposed to or infected by HIV-1, a prophylactically or therapeutically effective amount of a compound of formula I, as set forth in claims 1,2,3 or 4.

6. A pharmaceutical composition, suitable for preventing or treating HIV-1 infection, which comprises a prophylactically or therapeutically effective amount of a compound of formula I, as set forth in claims 1,2,3

or 4, and a pharmaceutically acceptable carrier.